# EUROPÄISCHE PATENTSCHRIFT

(11) **EP 2 212 081 B1**
(45) Veröffentlichungstag und Bekanntmachung des Hinweises auf die Patenterteilung: **04.04.2012**
(21) Anmeldenummer: 08848999.2
(22) Anmeldetag: 10.11.2008
(51) Int. Cl.: B29C 44/12, C08J 9/00, C08L 101/14

(54) **SUPERABSORBIERENDER SCHAUM MIT GRAFISCHEN ZEICHEN AN DER OBERFLÄCHE**
SUPERABSORBENT FOAM HAVING GRAPHICAL SYMBOLS ON ITS SURFACE
MOUSSE SUPER-ABSORBANTE AVEC DES SIGNES GRAPHIQUES SUR LA SURFACE

(30) Priorität: 15.11.2007 EP 07120800
(43) Veröffentlichungstag der Anmeldung: 04.08.2010
(73) Patentinhaber: BASF SE, 67056 Ludwigshafen (DE)
(72) Erfinder: ZIEMER, Antje, 68199 Mannheim (DE); BAUER, Ernst Jürgen, 67069 Ludwigshafen (DE)
(86) Internationale Anmeldenummer: PCT/EP2008/065195
(87) Internationale Veröffentlichungsnummer: WO 2009/062902

(56) Entgegenhaltungen:
- WO-A-94/25513
- WO-A-2006/106108
- GB-A- 1 244 086
- US-A1- 2005 250 866

## Beschreibung

Die Erfindung betrifft einen superabsorbierenden Schaum, der an der Oberfläche grafische Zeichen aufweist, sowie ein Verfahren zu seiner Herstellung und seine Verwendung.

Superabsorber sind bekannt. Für derartige Materialien sind auch Bezeichnungen wie "hochquellfähiges Polymer" "Hydrogel" (oft auch für die trockene Form verwendet), "Hydrogel bildendes Polymer", "Wasser absorbierendes Polymer", "absorbierendes gelbildendes Material", "quellfähiges Harz", "wasserabsorbierendes Harz" oder ähnliche gebräuchlich. Es handelt sich dabei um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure am weitesten verbreitet sind. Die wesentlichen Eigenschaften von Superabsorbern sind ihre Fähigkeiten, ein Vielfaches ihres Eigengewichts an wässrigen Flüssigkeiten zu absorbieren und die Flüssigkeit auch unter gewissem Druck nicht wieder abzugeben. Der Superabsorber, der meist in Form eines trockenen Pulvers eingesetzt wird, aber auch in Form von Schäumen bekannt ist, wandelt sich bei Flüssigkeitsaufnahme in ein Gel, bei der Aufnahme von Wasser entsprechend in ein Hydrogel (das in diesem Falle auch tatsächlich eines ist) um. Die Vernetzung ist für synthetische Superabsorber wesentlich und ein wichtiger Unterschied zu üblichen reinen Verdickern, da sie zur Unlöslichkeit der Polymeren in Wasser führt. Lösliche Substanzen wären als Superabsorber nicht brauchbar. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise die als wasserzurückhaltende Mittel im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

Superabsorbierende Schäume, also Wasser absorbierende Schäume auf Basis von vernetzten, Säuregruppen enthaltenden Monomeren oder auf Basis vernetzter basischer Polymere sind ebenfalls bekannt. Superabsorbierende Schäume können beispielsweise als Flüssigkeitsspeicherschicht ("Storage Layer") in Hygieneartikeln oder allgemein zum Aufsaugen, Weiterleiten oder Speichern von wässrigen Flüssigkeiten verwendet werden.

Auch Verfahren zur Herstellung von Superabsorbern sind bekannt. Superabsorber auf Basis von Acrylsäure, die auf dem Markt am gängigsten sind, werden durch radikalische Polymerisation von Acrylsäure in Gegenwart eines Vernetzers (dem "Innenvernetzer") hergestellt, wobei die Acrylsäure vor, nach oder teils vor, teils nach der Polymerisation zu einem gewissen Grad neutralisiert wird, üblicherweise durch Zugabe von Alkali, meist einer wässrigen Natriumhydroxidlösung. Der Innenvernetzer ist typischerweise eine Verbindung mit mindestens zwei polymerisierbaren Gruppen, die in verschiedene der bei der Polymerisation aus den Acrylsäure-Monomeren entstehenden Polymerketten einpolymerisiert werden und so die Polymerkette untereinander vernetzen. Das so gewonnene Polymergel wird zerkleinert (je nach verwendetem Polymerisationsreaktor kann dies gleichzeitig mit der Polymerisation erfolgen) und getrocknet. Das so gewonnene trockene Pulver (das "Grundpolymer" oder "Basispolymer") wird üblicherweise an der Oberfläche der Partikel nachvernetzt, indem es mit weiteren Vernetzern, die Verknüpfungen zwischen verschiedenen funktionellen Gruppen des Polymers herstellen können, wie etwa organischen Vernetzern oder mehrwertigen Kationen, beispielsweise Aluminium (meist als Aluminiumsulfat eingesetzt) umgesetzt wird, um eine gegenüber dem Partikelinneren stärker vernetzte Oberflächenschicht zu erzeugen.

Verfahren zur Herstellung superabsorbierender Schäume sind ebenfalls bekannt. Dabei wird üblicherweise eine das Monomer enthaltende Mischung aufgeschäumt. Dies kann durch mechanisches Dispergieren von Gasblasen erfolgen, beispielsweise durch Einschlagen von Gas oder Einpressen von Gas und Entspannung durch eine Düse, aber auch durch Zersetzung eines Gas bildenden Blähmittels in der Monomerlösung. Die so geschäumte Mischung wird dann polymerisiert und wahlweise nachbehandelt.

Frederic L. Buchholz und Andrew T. Graham (Hrsg.) geben in: "Modern Superabsorbent Polymer Technology", J. Wiley & Sons, New York, U.S.A. / Wiley-VCH, Weinheim, Germany, 1997, ISBN 0-471-19411-5, einen zusammenfassenden Überblick über bekannte Verfahren zur Herstellung von Superabsorbern und über Superabsorberschäume.

Beispiele superabsorbierender Schäume und Verfahren zu ihrer Herstellung sind etwa WO 97/17 397 A1, WO 97/31971 A1, WO 99/44648 A1 und WO 00/52087 A1. Diese Schäume werden durch Schäumen einer polymerisierbaren wässrigen Mischung, die zu mindestens 50 Mol-% neutralisierte, Säuregruppen enthaltende monoethylenisch ungesättigte Monomere, Vernetzer und mindestens ein Tensid enthält, und anschließendes Polymerisieren der geschäumten Mischung hergestellt. Das Schäumen der polymerisierbaren Mischung erfolgt durch Dispergieren von feinen Blasen eines gegenüber Radikalen inerten Gases oder durch Lösen eines solchen Gases unter erhöhtem Druck in der polymerisierbaren Mischung und Entspannen der Mischung. Der Wassergehalt der Schaumstoffe wird auf 1 bis 60 Gew.-% eingestellt. Die Schäume können wahlweise einer Oberflächennachvernetzung unterworfen werden, indem man einen Vernetzer auf das geschäumte Material sprüht oder den Schaum darin eintaucht und den mit Vernetzer beladenen Schaum auf eine höhere Temperatur erhitzt. Die Schäume werden z.B. in Hygieneartikeln zur Akquisition, Distribution und Speicherung von Körperflüssigkeiten verwendet. Carboxymethylcellulose, Hydroxyethylcellulose, Hydroxymethylcellulose, Hydroxypropylcellulose und Cellulosemischether werden in diesen Anmeldungen als Verdicker offenbart. Es ist auch bekannt, den Schaum durch Zusatz von Superabsorber-Feinteilchen zu verfestigen. Schäume werden oft durch Zugabe von Fasern gegen Bruch oder Riss stabilisiert.

Aus WO 03/06 6716 A1 sind Schaumstoffe aus wasserabsorbierenden basischen Polymeren bekannt, die durch Schäumen einer wässrigen Mischung, die mindestens ein basisches Polymer wie Polyvinylamin und mindestens einen Vernetzer wie Glycidylether enthält, und anschließendes Vernetzen der geschäumten Mischung erhältlich sind.

Aus WO 03/066717 A2 ist ein Verfahren bekannt, mit dem durch die Zugabe von aminogruppenhaltigen Polymeren die Nassfestigkeit von superabsorbierenden Schäumen erhöht und der Restmonomerengehalt erniedrigt wird.

Aus WO 2004/007598 A1 sind wasserabsorbierende Schäume bekannt, die an der Oberfläche fein verteiltes hydrophiles Siliziumdioxid und/oder einem oberflächenaktiven Stoff aufweisen. WO 2006/106108 A1 offenbart Schäume, die durch Behandlung mit einem Quellverzögerungsmittel langsamer absorbieren als konventionelle Schäume.

Aus WO 2004/035668 A2 sind wasserabsorbierende Schäume bekannt, die superabsorbierende Fasern oder Fruchtfasern, insbesondere Apfelfasern enthalten. In WO 2006/094977 A2 sind wasserabsorbierende Schäume beschrieben, die Holzfasern oder Altpapierfasern enthalten.

Es ist ferner bekannt, partikulären Superabsorbern anorganische Feststoffe als Füllstoffe beizumischen. Insbesondere werden dazu Tonmineralien verwendet. GB 2 082 614 A offenbart eine Abmischung aus Superabsorberpulver und einem aus unvernetzten Cellulosederivaten, Stärke, bestimmten Tonen und Mineralien oder deren Gemischen gewählten Füllstoff. Das Gemisch weist eine höhere Absorptionsfähigkeit als die berechnete Summe der Bestandteile auf. US 4 500 670 lehrt ein Gemisch aus Superabsorber und anorganischen, wasserunlöslichen Pulvern, in dem das anorganische Pulver die Steifigkeit des gequollenen Gels verbessert. In dem von US 4 735 987 gelehrten Suspensionspolymerisationsverfahren wird ein stark expandierender und gasdurchlässiger Superabsorber erzeugt, indem Superabsorberpartikel in Suspension untereinander vernetzt werden, wobei die Anwesenheit eines anorganischen Füllstoffs US 4 914 066 offenbart Formkörper aus Bentonit mit 0,5 bis 15 Gew.-% Superabsorber.

Nach der Lehre von WO 91/12 029 A1, WO 91/12 031 A1 oder EP 799 861 A1 werden wasserunlösliche Zeolithe oder Aktivkohle als Zusatz zu Superabsorbern verwendet, um Bildung unangenehmer Gerüche einzudämmen. Nach WO 01/13 965 A1 werden dazu siliziumreiche Zeolithe verwendet.

US 5 419 956 offenbart absorbierende Artikel, die Superabsorber enthalten, dem zur Verbesserung der Flüssigkeitsverteilung anorganische Pulver wie Siliciumdioxid, Aluminiumoxid, Titandioxid oder Tone, beispielsweise Kaolin und Montmorillonit zugesetzt werden. WO 01/68 156 A1 beschreibt Superabsorber, denen sowohl zur Verbesserung der Flüssigkeitsweiterleitung als auch zur Bindung unangenehmer Gerüche Alumosilikate, insbesondere solche mit Schichtstrukturen wie Saponit oder Montmorillonit zugegeben werden.

US 3 900 378 lehrt, bei der Herstellung eines Superabsorbers durch Vernetzung löslicher Polymere mittels ionisierender Strahlung einen Füllstoff als Dispergator der Polymerpartikel zuzusetzen. Als Beispiele für Füllstoffe sind unter anderem auch Mineralien wie Perlit, Kieselgur, Tone, Flugasche und Magnesiumsilikate genannt. Nach der Lehre von US 5 733 576 können derartige Füllstoffe bei der Herstellung eines Superabsorbers, der ein Gemisch aus vernetztem Polyacrylat und Polysaccharid ist, zugegeben werden.

US 6 124 391 offenbart die Verwendung von anorganischen Pulvern, insbesondere Tonen wie Kaolin, als Mittel gegen die Verbackungsneigung von Superabsorbern.

WO 00/72 958 A1 beschreibt die Verwendung von Tonen als synergistische Füllstoffe von Superabsorbern. WO 01/32 117 A1 lehrt die Verwendung von Hydrotalcit als basischen Füllstoff in einem leicht sauren Superabsorber, um dessen Toleranz gegenüber Natriumchlorid zu steigern.

Der Erfindung liegt die Aufgabe zugrunde, einen superabsorbierenden Schaum zu finden, der an seiner Oberfläche grafische Zeichen aufweist, sowie ein Verfahren zu dessen Herstellung. Dem gemäß wurde ein superabsorbierender Schaum gefunden, der mindestens 1 Gew.-%, bezogen auf das Gesamtgewicht des trockenen Schaums, anorganischen pulverförmigen Feststoffs enthält und an mindestens einer seiner Oberflächen grafische Zeichen trägt, der erhältlich ist durch ein Verfahren, bei dem eine geschäumte wässrige Mischung, die
mindestens 1 Gew.-%, bezogen auf das Gesamtgewicht des trockenen Schaums, anorganischen pulverförmigen Feststoffs
sowie polymerisier- und vernetzbare, wahlweise (teil-)neutralisierte, Säuregruppen enthaltende monoethylenisch ungesättigte Monomere oder mindestens ein vernetzbares basisches Polymer,
sowie Vernetzer, anorganischen pulverförmigen Füllstoff und mindestens ein Tensid
enthält, in einer Form polymerisiert wird, die auf mindestens einer Innenfläche grafische Zeichen trägt.

Unter "Oberfläche" des Schaums wird im Rahmen dieser Erfindung die geometrische Oberfläche eines Schaumformkörpers verstanden, nicht die gesamte innere Oberfläche aller Poren des Schaums, wie sie beispielsweise durch die bekannten Verfahren der Messung von Adsorptionsisothermen bestimmt werden könnte.

Unter grafischen Zeichen werden alle Arten von Zeichen und Mustern verstanden, die auf einer im Wesentlichen ebenen oder ebenen Oberfläche durch Punkte, Linien, Flächen, Füllmuster oder sonstige grafische Gestaltungselemente darstellbar sind, insbesondere Schriftzeichen, Buchstaben, Zeichnungen, geometrische, gegenständliche oder abstrakte Muster und Abbildungen. Eine echte dreidimensionale Gestaltung eines Schaumformkörpers, die in einfacher Weise durch entsprechende dreidimensionale geometrische Ausbildung der Form erzeugt werden kann, in der der Schaum polymerisiert wird, ist damit nicht gemeint. Mit einer im Wesentlichen ebenen Oberfläche ist gemeint, dass die Oberfläche optisch einen im Wesentlichen flächigen Eindruck vermittelt, aber die grafischen Zeichen etwas hinter die Oberfläche des Schaums zurücktreten oder daraus hervortreten können. Die Zeichen können also durchaus fühlbar sein, auch wenn die Dreidimensionalität ebenso wie bei Druckverfahren auf Papier typischerweise minimal ist.

Superabsorbierende Schäume (oft auch einfach "Superabsorberschäume" genannt) sind aus dem Stand der Technik bekannt. Unter superabsorbierenden Schaum gemäß der vorliegenden Erfindung wird ein Schaum verstanden, der eine Zentrifugenretentionskapazität ("CRC", "Centrifuge Retention Capacity", Messmethode unten im Abschnitt "Bestimmungsmethoden" beschrieben) von mindestens 3 g/g, bevorzugt mindestens 4 g/g, besonders bevorzugt mindestens 5 g/g, insbesondere mindestens 6 g/g aufweist.

Die erfindungsgemäßen Superabsorberschäume enthalten mindestens 1 Gew.-%, bezogen auf das Gesamtgewicht des trockenen Schaums, anorganischen pulverförmigen Feststoff. Im Zusammenhang mit Superabsorberschäumen wird dieser oft auch "Füllstoff" genannt. Füllstoffe enthaltende Superabsorberschäume sind bekannt.

Superabsorberschäume haben wie partikuläre Superabsorber auch meist einen endlichen Wassergehalt. Eine Trocknung auf einen nicht mehr messbaren Restwassergehalt ist aufwendig und Superabsorber zieht Feuchtigkeit aus der Umgebung an. Oft wird der Wassergehalt im Schaum absichtlich auf einen bestimmten gewünschten Wert eingestellt, im allgemeinen auf mindestens 1 Gew.-%, vorzugsweise mindestens 2 Gew.-% und in besonders bevorzugter Form auf mindestens 3 Gew.-%, jeweils bezogen auf das Gesamtgewicht des befeuchteten Schaums. Eine Obergrenze für den Wassergehalt ist letztlich allein durch wirtschaftliche Erwägungen im Hinblick auf die typische Verwendung der Schäume zur Absorption von Flüssigkeiten gegeben, da mit steigendem Wassergehalt die Absorptionskapazität entsprechend sinkt. Meist wird daher ein Wassergehalt im Schaum - vor dessen Anwendung zur Flüssigkeitsabsorption - von nicht mehr als 50 Gew.-%, vorzugsweise nicht mehr als 30 Gew.-% und in besonders bevorzugter Form von nicht mehr als 20 Gew.-% eingestellt, jeweils bezogen auf das Gesamtgewicht des befeuchteten Schaums. Typische Wassergehalte von Schäumen betragen beispielsweise 5 Gew.-% oder 10 Gew.-%, jeweils bezogen auf das Gesamtgewicht des befeuchteten Schaums. Superabsorber fühlen sich jedoch auch bei hohen Wassergehalten trocken an. Die Wasseraufnahme ist reversibel. Um eine definierte Basis für Berechnung prozentualer Anteile zu schaffen, werden Mengenangaben im Rahmen dieser Anmeldung, sofern nicht anders angegeben, auf einen trockenen Superabsorberschaum bezogen, also einen Superabsorberschaum, der kein Wasser enthält. Für einen realen Schaum, der einen bestimmten Wassergehalt aufweist, ist entsprechend umzurechnen. Der Wassergehalt (auch "Feuchtigkeitsgehalt" oder "Restfeuchte" genannt) wird nach der unten unter "Bestimmungsmethoden" angegebenen Methode bestimmt.

Angaben in Gew.-% sind im Rahmen dieser Erfindung stets - auch für andere Anteile als Wasser - auf das Gesamtgewicht des trockenen Superabsorberschaums bezogen, sofern im Einzelfall nicht ausdrücklich anders angegeben.

Der anorganische pulverförmige Feststoff ist ein Füllstoff, dessen Anwesenheit für den Erfolg des erfindungsgemäßen Verfahrens und die Eignung einer Monomermischung, bei der Polymerisation grafische Zeichen an der Oberfläche auszubilden, wesentlich ist. Im Prinzip ist jedes anorganische Pulver geeignet.

Die Größe der einzelnen Partikel im Pulver liegt typischerweise bei im Allgemeinen mindestens 1 µm, vorzugsweise mindestens 5 µm und in besonders bevorzugter Form mindestens 10 µm sowie im Allgemeinen höchstens 1 000 µm, vorzugsweise höchstens 900 µm und in besonders bevorzugter Form höchstens 850 µm. Die Pulverpartikel können auch Aggregate oder Agglomerate aus kleineren Primärpartikeln sein. Es ist ebenso möglich, Agglomerate einzusetzen, die erst bei der Herstellung der Monomermischung zu kleineren Partikeln zerfallen, etwa um Probleme mit Staubbildung oder Bildung lungengängiger Stäube zu vermeiden.

Das anorganische Pulver ist ein partikelförmiger Feststoff. Beispiele solcher Feststoffe sind Oxide, Tone, Zeolithe, anorganische Pigmente, Mineralien oder allgemein feste, chemisch inerte (d.h. die Polymerisation sowie die Anwendung des Schaums nicht wesentlich beeinträchtigende) Stoffe.

Geeignete Oxide sind die Metalloxide der Gruppen 2 bis 14 des Periodensystems der Elemente einschließlich der Lanthaniden und Actiniden. Beispiele besonders geeigneter Metalloxide sind Magnesiumoxid, Calciumoxid, Strontiumoxid und Bariumoxid, Titandioxid, Zirkondioxid, Vanadiumoxid, Chromoxid, Molybdänoxid und Wolframoxid, Manganoxid, Eisenoxid, Kobaltoxid, Nickeloxid, Kupferoxid, Zinkoxid, Boroxid, Aluminiumoxid, Siliciumdioxid, Zinnoxid, Bleioxid, Lanthanoxid oder Ceroxid. Zur Klarstellung: Durch die Verwendung dieser gebräuchlichen Namen soll keine zwingende Aussage über die Wertigkeit des Metalls im Oxid, d.h. über die stöchiometrische Zusammensetzung des Oxids getroffen werden. Sofern von einem Element mehrere Oxide bekannt sind, ist im Allgemeinen die Verwendung aller bekannten Oxide möglich. Die Auswahl wird nach für den Einzelfall spezifischen Erwägungen, etwa nach den Kosten eines Oxids, seiner Toxizität, Stabilität oder auch Farbe getroffen. Beispiele sehr gut geeigneter Oxide sind Titandioxid, insbesondere in den Modifikationen Rutil oder Anatas, oder durch Fällung oder pyrolytisch hergestelltes Siliciumdioxid.

Tone sind mit Wasser quellbare Silikat- oder Alumosilikatmineralien, die typischerweise als Sedimentgestein abgebaut und teilweise nachbehandelt werden. Sie können allerdings auch synthetisch hergestellt werden. Beispiele sind insbesondere Kaolinit, insbesondere in der Form von Kaolin, Illit, Attapulgit (anderer Name: Palygorskit), Sepiolith, Montmorillonit, insbesondere in der Form von Bentonit, Pyrophyllit, Saponit oder Talk (anderer Name: Talkum). Auch andere Schichtsilikate oder-alumosilikate können verwendet werden, beispielsweise Vermiculit oder Hydrotalcit.

Beispiele sonstiger verwendbarer anorganischer Feststoffe sind Sulfate wie Magnesium- oder Bariumsulfat, Carbonate wie Calcium- oder Magnesiumcarbonat oder Dolomit, Silikate wie Calcium- oder Magnesiumsilikat, Carbide wie Perlit oder Siliciumkarbid, Diatomeenerde oder Flugasche.

Es können auch Gemische aus zwei oder mehreren dieser Feststoffe eingesetzt werden.

Manche Feststoffe, bei der Herstellung von Superabsorberschäumen aus einer Acrylsäure enthaltenden Monomermischung insbesondere durch Säure zersetzbare Carbonate wie Calciumcarbonat, können gleichzeitig auch zur Schaumbildung eingesetzt werden. In diesem Fall ist darauf zu achten, dass die Reaktionsparameter und die Zusammensetzung der Stoffmischung so gewählt werden, dass trotz der Zersetzung des Carbonats unter Schaumbildung eine ausreichende Menge partikelförmigen Feststoffs verbleibt.

Der Gehalt des superabsorbierenden Schaums an anorganischem Pulver ist im Allgemeinen mindestens 1 Gew.-%, vorzugsweise mindestens 2 Gew.-% und in besonders bevorzugter Form mindestens 5 Gew.-% sowie im Allgemeinen höchstens 50 Gew.-%, vorzugsweise höchstens 40 Gew.-% und in besonders bevorzugter Form höchstens 20 Gew.-%. Diese Obergrenze ist allerdings weniger von einer etwaigen Beeinflussung der Fähigkeit des Schaums zur Ausbildung grafischer Zeichen bestimmt als von der gewünschten Absorptionsfähigkeit des Schaums für Flüssigkeiten, die durch nicht superabsorbierende Anteile im Schaum naturgemäß gesenkt wird. Falls vergleichsweise niedrige Absorptionsfähigkeit des Schaums toleriert werden kann oder sogar gewünscht ist, kann der Anteil des anorganischen Pulvers auch über den angegebenen Obergrenzen liegen. Der für die Fähigkeit zur Ausbildung grafischer Zeichen optimale Pulvergehalt des Schaums ist vom konkreten Pulver abhängig und mit wenigen Routineversuchen leicht zu ermitteln. Beispielsweise ist die Fähigkeit eines Kaolin enthaltenden Schaums zur Ausbildung grafischer Zeichen ab einem Kaolingehalt von mindestens 10 Gew.-%, noch besser bei mindestens 20 Gew.-% optimal, bei einem Talkum enthaltenden Schaum bei einem Talkumgehalt von mindestens 20 Gew.-%, noch besser bei mindestens 40 Gew.-% und bei einem Titandioxid enthaltenden Schaum bei einem Titandioxidgehalt von mindestens 1 Gew.-%, noch besser bei mindestens 5 Gew.-%.

Die erfindungsgemäßen superabsorbierenden Schäume sind in bequemer Weise erhältlich durch Schäumen einer wässrigen Mischung, die neben polymerisierbaren und vernetzbaren, Säuregruppen enthaltenden monoethylenisch ungesättigten Monomeren, die wahlweise (teil-)neutralisiert sind, Vernetzer, den anorganischen pulverförmigen Feststoff und mindestens ein Tensid sowie wahlweise Zusätze oder Hilfsstoffe wie Lösevermittler, Verdicker, Stabilisatoren, Füllstoffe, Fasern und/oder Zellkeimbildner enthält, und anschließendes Polymerisieren und/oder Vernetzen der geschäumten Mischung.

Außerdem können die superabsorbierenden Schäume in bequemer Weise hergestellt werden, indem mindestens ein vernetzbares basisches Polymer, Vernetzer, der anorganische pulverförmige Feststoff und mindestens ein Tensid sowie wahlweise Zusätze oder Hilfsstoffe wie Lösevermittler, Verdicker, Stabilisatoren, Füllstoffe, Fasern und/oder Zellkeimbildner als Mischung geschäumt werden, und anschließend die in der geschäumten Mischung enthaltenen basischen Polymeren unter Bildung eines schaumförmigen Hydrogels vernetzt werden.

Der Schaum wird wahlweise anschließend mit einem Komplexierungs- und/oder Quellverzögerungsmittel behandelt. Der Einfachheit halber wird bei der Beschreibung von Maßnahmen oder Eigenschaften, die nicht spezifisch für Mischungen sind, die vernetzbare basische Polymere oder polymerisierbare und vernetzbare, Säuregruppen tragenden Monomere enthalten, auch für Mischungen, die zwar vernetzbare basische Polymere, aber keine polymerisierbaren und vernetzbaren, Säuregruppen tragenden Monomere enthalten, auch der Begriff "polymerisierbare wässrige Mischung" oder auch einfach "Monomermischung" verwendet.

Zur Erzeugung grafischer Zeichen auf der Oberfläche des superabsorbierenden Schaums wird dieser in einer Form polymerisiert, die auf mindestens einer Innenfläche grafische Zeichen aufweist. Diese grafischen Zeichen sind auf dieser Innenfläche durch eine andere chemische Zusammensetzung oder eine andere physikalische Oberflächenbeschaffenheit als die übrigen Bereiche der Innenfläche von diesen unterschieden. Die Innenfläche der Form ist die Fläche der Form, deren Oberfläche bei der Polymerisation der Monomermischung zum Superabsorberschaum mit der Monomermischung oder dem Schaum in Kontakt steht. Als "Form" ist jede räumliche Begrenzung des Volumens zu verstehen, in dem die Polymerisation stattfindet. Dies muss nicht notwendigerweise eine geschlossene Form sein, sondern kann auch ein temporäres Trägermaterial sein, wie es etwa in einem Bandreaktor zur Polymerisation der Fall ist. Die "Innenfläche" der Form ist dabei jede Fläche, die mit polymerisierendem Schaum in Kontakt steht und diesem dadurch an der Kontaktfläche eine bestimmte geometrische Form verleiht.

Es steht zu vermuten, dass diese auf der Innenfläche der Form ausgebildeten grafischen Zeichen lokal begrenzt die Polymerisation des Schaums so beeinflussen, dass korrespondierende grafische Zeichen sichtbar auf dem Schaum entstehen, die bei der Ausformung und Durchpolymerisation des Superabsorberschaums erhalten bleiben. Durch Farbe oder Lack auf die Form aufgebrachte grafische Zeichen bilden sich auf dem Schaum jedenfalls auch dann ab, wenn sie nicht auf ihn abfärben.

In einer einfachen Ausführungsform des erfindungsgemäßen Verfahrens wird die Monomermischung in einer Form polymerisiert, die an mindestens einer Innenfläche grafische Zeichen aufweist, die durch Stellen der Innenfläche mit einer anderen Rauhigkeit als die restliche Innenfläche gebildet werden, also eine andere physikalische Oberflächenbeschaffenheit haben.

In einer anderen Ausführungsform wird die Monomermischung in einer Form polymerisiert, die an mindestens einer Innenfläche grafische Zeichen aufweist, die durch Auftrag von Farbe oder Lack erzeugt wurden, also eine andere chemische Zusammensetzung der Oberfläche aufweisen als die übrige Innenfläche der Form. In einer weiteren Ausführungsform wird die Monomermischung in einer Form polymerisiert, die an mindestens einer Innenfläche grafische Zeichen aufweist, die durch chemische Veränderung der Oberfläche erzeugt wurden, etwa durch Ätzung, aber auch durch physikalische oder physikalisch-chemische Verfahren wie lonenimplantierung oder Abscheidung chemischer Verbindungen aus der Gasphase.

In einer bequemen Ausführungsform des erfindungsgemäßen Verfahrens wird die Innenseite der Form mit Farbe oder Lack bedruckt oder bemalt. Die aufgedruckten grafischen Zeichen bilden sich entsprechend auf dem Schaum ab. Typischerweise findet dabei keine Farbübertragung von der Form auf den Schaum statt. Zur Herstellung von vergleichsweise dünnem Rollgut kann der Schaum beispielsweise zwischen zwei Folien polymerisiert werden, von denen eine oder beide bedruckt oder bemalt sind. Die Polymerisation kann dabei kontinuierlich oder diskontinuierlich erfolgen.

In einer beispielhaften Ausführungsform der Erfindung schäumt man eine wässrige Mischung, die
a) 10 bis 80 Gew.-% Säuregruppen enthaltende monoethylenisch ungesättigte Monomere, die zu mindestens 50 Mol-% neutralisiert sind, oder ein basisches vernetzbares Polymer;
b) bei Verwendung Säuregruppen tragender monoethylenisch ungesättiger Monomere, wahlweise zusätzlich bis zu 50 Gew.-% andere monoethylenisch ungesättigte Monomere,
c) 0,001 bis 10 Gew.-% Vernetzer,
d) bei Verwendung monoethylenisch ungesättiger Monomere, zusätzlich Initiatoren,
e) 0,1 bis 20 Gew.-% mindestens eines Tensids,
f) wahlweise einen Lösevermittler,
g) wahlweise Verdicker, Schaumstabilisatoren, Polymerisationsregler, Füllstoffe, Fasern und/oder Zellkeimbildner
   enthält, jeweils bezogen auf die Gesamtmenge der wässrigen Mischung, sowie
h) 1 bis 50 Gew.-% anorganischen Feststoff, bezogen auf das Gewicht des fertigen trockenen Schaums,
wobei die Gesamtmenge der einzelnen Komponenten der Mischung einschließlich des Wassers sich zu 100 Gew.-% addieren.

Der anorganische Feststoff ist in der wässrigen Mischung üblicherweise zu mindestens 0,5 Gew.-% und höchstens 30 Gew.-% enthalten.

Das Schäumen der wässrigen Mischungen kann beispielsweise dadurch erfolgen, dass man feine Blasen eines gegenüber Radikalen inerten Gases in der Mischung dispergiert oder ein solches Gas unter einem Druck von 2 bis 400 bar in der vernetzbaren Mischung löst und sie anschließend auf Atmosphärendruck entspannt. Man erhält einen fließfähigen Schaum, der in Formen eingefüllt oder auf einem Band ausgehärtet werden kann. Das Aushärten erfolgt im Fall des Einsatzes von Säuregruppen enthaltenden Monomeren, wahlweise anderen monoethylenisch ungesättigten Monomeren und Vernetzern durch Polymerisation und im Fall des Einsatzes von basischen Polymeren unter Vernetzung.

Superabsorberschäume auf Basis von vernetzten, Säuregruppen enthaltenden Polymerisaten.

Superabsorberschäume auf Basis von vernetzten, Säuregruppen enthaltenden Polymerisaten sind bekannt, unter anderem aus den zum Stand der Technik genannten Schriften EP 858 478 B1, Seite 2, Zeile 55 bis Seite 18, Zeile 22, WO 99/44 648 A1 und WO 00/52 087 A1, Seite 5, Zeile 23 bis Seite 41, Zeile 18, auf die alle hier ausdrücklich Bezug genommen wird. Als Säuregruppen enthaltende monoethylenische ungesättigte Monomere kommen die Monomere und Monomermischungen in Frage, die zur Herstellung von granulärem Superabsorber verwendet werden. Bevorzugtes Monomer ist Acrylsäure und deren Salze. Weitere monoethylenisch ungesättigte Monomere sind ebenfalls aus der Literatur bezüglich Herstellung von granulärem Superabsorber bekannt.

In einer Ausführungsform werden Schäume aus wasserabsorbierenden sauren Polymeren verwendet. Als wasserabsorbierende saure Polymere, die nachfolgend auch als saure Superabsorber bezeichnet werden, können sämtliche Hydrogele eingesetzt werden, die z.B. in der WO 00/63 295 A1, Seite 2, Zeile 27 bis Seite 9, Zeile 16 beschrieben sind. Hierbei handelt es sich im Wesentlichen um schwach vernetzte Polymere von sauren Monomeren, die in zumindest partiell neutralisierter Form ein hohes Wasseraufnahmevermögen besitzen. Beispiele für solche jeweils geringfügig vernetzten Polymerisate sind vernetzte Polyacrylsäuren, vernetzte, hydrolysierte Pfropfpolymere von Acrylnitril auf Stärke, vernetzte Pfropfpolymerisate von Acrylsäure auf Stärke, hydrolysierte, vernetzte Copolymere aus Vinylacetat und Acrylsäureestern, vernetzte Polyacrylamide, hydrolysierte, vernetzte Polyacrylamide, vernetzte Copolymerisate aus Ethylen und Maleinsäureanhydrid, vernetzte Copolymerisate aus Isobutylen und Maleinsäureandhydrid, vernetzte Polyvinylsulfonsäuren, vernetzte Polyvinylphosphonsäuren und vernetztes sulfoniertes Poylstyrol. Vorzugsweise verwendet man als saure Superabsorber Polymerisate von (teil-)neutralisierten, geringfügig vernetzten Polyacrylsäuren. Die (Teil-)neutralisation der Säuregruppen der sauren Superabsorber erfolgt vorzugsweise mit Natronlauge, Natriumhydrogencarbonat oder Natriumcarbonat. Die Neutralisation kann jedoch auch mit Kalilauge, Ammoniak, Aminen oder Alkanolaminen wie Ethanolamin, Diethanolamin oder Triethanolamin vorgenommen werden.

Saure Superabsorber sind aus den obengenannten Literaturstellen bekannt, unter anderem aus WO 00/63 295 A1, Seite 2, Zeile 27 bis Seite 9, Zeile 16, auf die ausdrücklich Bezug genommen wird. Sie können wahlweise oberflächennachvernetzt werden, dazu werden beispielsweise geringfügig vernetzte Polyacrylsäuren mit Verbindungen umgesetzt, die mindestens zwei gegenüber Carboxylgruppen reaktive Gruppen aufweisen. Hierbei handelt es sich um bekannte Vernetzer. Von besonderem Interesse für die Anwendung als Oberflächennachvernetzer sind beispielsweise mehrwertige Alkohole wie Propylenglykol, Butandiol-1,4 oder Hexandiol-1,6 und Glycidylether von Ethylenglykol und Polyethylenglykolen mit Molmassen von 200 bis 1500, vorzugsweise 300 bis 400 Dalton, und vollständig mit Acrylsäure oder Methacrylsäure veresterte Umsetzungsprodukte von Trimethylolpropan, von Umsetzungsprodukten aus Trimethylolpropan und Ethylenoxid im Molverhältnis 1 : 1 bis 25, vorzugsweise 1 : 3 bis 15 sowie von Umsetzungsprodukten von Pentaerythrit mit Ethylenoxid im Molverhältnis 1 : 30, vorzugsweise 1 : 4 bis 20. Die Nachvernetzung der Oberfläche der anionischen Superabsorber wird, sofern sie durchgeführt wird, beispielsweise bei Temperaturen bis zu 220°C, z.B. vorzugsweise bei 120 bis 190°C durchgeführt.

### Vernetzbare basische Polymere

Die Herstellung von Superabsorberschäumen aus vernetzbaren basischen Polymeren ist bekannt, unter Anderem aus WO 03/066 716 A1, auf die hier ausdrücklich Bezug genommen wird.

### Weitere Monomere

Eine polymerisierbare wässrige Mischung kann neben Säuregruppen tragenden Monomeren auch weitere ethylenisch ungesättigte Monomere enthalten. Geeignete ethylenisch ungesättigte Monomere sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

### Vernetzer

Hier, im Zusammenhang mit der Polymerisation einer Monomermischung (oder alternativ der Vernetzung von basischen Polymeren) ist unter "Vernetzer" der bei granulärem Superabsorber typischerweise "Innenvernetzer" genannte Vernetzer zu verstehen, der die Polymerketten des Superabsorbers miteinander vernetzt, wobei typischerweise - abgesehen vom Effekt von Diffusion des Vernetzers während der vernetzenden Polymerisation oder der Vernetzung der Polymerketten - ein im Wesentlichen einheitlicher Vernetzungsgrad über das Volumen des Superabsorbers erreicht wird. Superabsorber werden jedoch oft auch an der Oberfläche mit einem Oberflächenvernetzer nachvernetzt, wobei der Vernetzungsgrad an der Oberfläche gegenüber dem im Volumen erhöht wird.

Vernetzer für basische Polymere zur Herstellung superabsorbierender Schäume sind wie diese selbst bekannt, unter anderem aus WO 03/066 716 A1, auf die hier ausdrücklich Bezug genommen wird.

Vernetzer für Polymere auf Basis Säuregruppen tragender Monomere sind ebenfalls bekannt. Eingesetzt werden die auch für die Herstellung granulärer Superabsorber bekannten Vernetzer. Diese sind Verbindungen mit mindestens zwei polymerisierbaren Gruppen, die in das Polymernetzwerk radikalisch einpolymerisiert werden können. Geeignete Vernetzer sind beispielsweise Ethylenglykoldimethacrylat, Diethylenglykoldiacrylat, Allylmethacrylat, Trimethylolpropantriacrylat, Triallylamin, Tetraallyloxyethan, Di- und Triacrylate, gemischte Acrylate, die neben Acrylatgruppen weitere ethylenisch ungesättigte Gruppen enthalten oder Vernetzermischungen.

Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glycerindi- und Glycerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer sind jedoch Di- und Triacrylate des 3- bis 15-fach ethoxylierten Glycerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glycerins oder Trimethylolpropans, des 3-fach propoxylierten Glycerins oder Trimethylolpropans, sowie des 3-fach gemischt ethoxylierten oder propoxylierten Glycerins oder Trimethylolpropans, des 15-fach ethoxylierten Glycerins oder Trimethylolpropans, sowie des 40-fach ethoxylierten Glycerins, Trimethylolethans oder Trimethylolpropans.

Ganz besonders bevorzugte Vernetzer sind die mit Acrylsäure oder Methacrylsäure zu Di- oder Triacrylaten veresterten mehrfach ethoxylierten und/oder propoxylierten Glycerine, wie sie beispielsweise in WO 03/104 301 A1 beschrieben sind. Besonders vorteilhaft sind Di- und/oder Triacrylate des 3- bis 10-fach ethoxylierten Glycerins. Ganz besonders bevorzugt sind Di- oder Triacrylate des 1- bis 5- fach ethoxylierten und/oder propoxylierten Glycerins. Am meisten bevorzugt sind die Triacrylate des 3- bis 5-fach ethoxylierten und/oder propoxylierten Glycerins.

### Initiatoren

Als Initiatoren der Polymerisationsreaktion können sämtliche unter den Polymerisationsbedingungen in Radikale zerfallende Verbindungen eingesetzt werden, z. B. Peroxide, Hydroperoxide, Wasserstoffperoxid, Persulfate, Azoverbindungen und die sogenannten Redoxinitiatoren, sowie jede andere bekannte Methode zur Erzeugung von Radikalen, beispielsweise hochenergetische Strahlung wie etwa UV-Licht. Bevorzugt ist der Einsatz von wasserlöslichen Initiatoren oder UV-Licht. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, Di-(2-ethylhexyl)peroxidicarbonat, Dicyclohexylperoxidicarbonat, Di-(4-tert.-butylcyclohexyl)peroxidicarbonat, Dimyristilperoxidicarbonat, Diacetylperoxi-dicarbonat, Allylperester, Cumylperoxyneodecanoat, tert.-Butylper-3,5,5-tri-methylhexanoat, Acetylcyclohexylsulfonylperoxid, Dilaurylperoxid, Dibenzoylperoxid und tert.-Amylperneodekanoat. Weitere geeignete Polymerisationsinitiatoren sind Azostarter, z. B. 2,2'-Azobis-(2-amidinopropan)dihydrochlorid, 2,2'-Azobis-(N,N-dimethylen)isobutyramidin-dihydrochlorid, 2-(Carbamoylazo)isobutyronitril, 2,2'-Azobis(2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z. B. in Mengen von im Allgemeinen mindestens 0,01 Mol-%, vorzugsweise mindestens 0,05 sowie in besonders bevorzugter Weise mindestens 1 Mol-% sowie im Allgemeinen höchstens 5, vorzugsweise höchstens 2 Mol-%, bezogen auf die zu polymerisierenden Monomere.

Die Redoxinitiatoren enthalten als oxidierende Komponente mindestens eine der oben angegebenen Perverbindungen und eine reduzierende Komponente, beispielsweise Ascorbinsäure, Glukose, Sorbose, Ammonium- oder Alkalimetallhydrogensulfit, -sulfit, -thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxinitiators Ascorbinsäure, Natriumsulfit oder Natriumpyrosulfit. Bezogen auf die bei der Polymerisation eingesetzte Menge an Monomeren verwendet man im Allgemeinen mindestens 3 · 10⁻⁶, vorzugsweise mindestens 1 · 10⁻⁵ bis 1 Mol-% der reduzierenden Komponente des Redoxinitiators und im Allgemeinen mindestens 1 · 10⁻⁵, vorzugsweise mindestens 1 · 10⁻³ bis 5 Mol-% der oxidierenden Komponente. Anstelle der oxidierenden Komponente oder zusätzlich kann man auch einen oder mehrere wasserlösliche Azostarter verwenden.

In einer Ausführungsform der Erfindung wird ein Redoxinitiator bestehend aus Wasserstoffperoxid, Natriumperoxodisulfat und Ascorbinsäure eingesetzt. Beispielsweise werden diese Komponenten in den Konzentrationen 1 · 10⁻² Mol-% Wasserstoffperoxid, 0,084 Mol-% Natriumperoxodisulfat und 2,5 · 10⁻³ Mol-% Ascorbinsäure, bezogen auf die Monomere eingesetzt.

Die Polymerisation kann allerdings auch in Abwesenheit von Initiatoren der obengenannten Art durch Einwirkung energiereicher Strahlung in Gegenwart von Photoinitiatoren ausgelöst werden. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethyl-amino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-% bezogen auf die zu polymerisierenden Monomeren angewendet.

Die wässrige Monomerlösung kann den Initiator gelöst oder dispergiert enthalten. Die Initiatoren können dem Polymerisationsreaktor jedoch auch getrennt von der Monomerlösung zugeführt werden.

### Tenside

Die polymerisierbaren bzw. vernetzbaren wässrigen Mischungen enthalten als weitere Komponente 0,1 bis 20 Gew.-% mindestens eines Tensids. Die Tenside sind für die Herstellung und die Stabilisierung des Schaums von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Verwendbare nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für einsetzbare nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additionsprodukte von 80 Mol Ethylenoxid an 1 Mol Talgfettalkohol. Weitere verwendbare handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere verwendbare handelsübliche nichtionische Tenside werden durch Ethoxylierung von Rizinusöl erhalten. Pro Mol Rizinusöl werden beispielsweise 12 bis 80 Mol Ethylenoxid angelagert. Weitere einsetzbare handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines C₁₃/C₁₅-Oxoalkohols, oder die Umsetzungsprodukte von 7 bis 8 Mol Ethylenoxid an 1 Mol eines C₁₃/C₁₅-Oxoalkohols. Weitere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines C₁₂- bis C₁₈-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten C₁₃/C₁₅-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche einsetzbare anionische Tenside. Weitere geeignete anionische Tenside sind Schwefelsäurehalbester von C₁₃/C₁₅-Oxoalkoholen, Paraffinsulfonsäuren wie C₁₅-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure sowie Fettalkoholphosphate wie C₁₅/C₁₈-Fettalkoholphosphat. Die polymerisierbare wässrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt der wässrigen Mischung beträgt vorzugsweise 0,5 bis 10 Gew.-%. In den meisten Fällen weisen die wässrigen Mischungen einen Tensidgehalt von 1,5 bis 8 Gew.-% auf.

### Lösevermittler

Die vernetzbaren wässrigen Mischungen können wahlweise als weitere Komponente mindestens einen Lösevermittler enthalten. Hierunter sollen mit Wasser mischbare organische Lösemittel verstanden werden, z.B. Dimethylsulfoxid, Dimethylformamid, N-Methylpyrrolidon, einwertige Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glycerinmonomethylether.

Die wässrigen Mischungen enthalten 0 bis 50 Gew.-% mindestens eines Lösevermittlers. Falls Lösevermittler eingesetzt werden, beträgt ihr Gehalt in der wässrigen Mischung vorzugsweise 1 bis 25 Gew.-%.

### Verdicker, Schaumstabilisatoren, Fasern, Zellkeimbildner

Die vernetzbare wässrige Mischung kann wahlweise Verdicker, Schaumstabilisatoren, Fasern und/oder Zellkeimbildner enthalten. Verdicker werden beispielsweise zur Optimierung der Schaumstruktur und zur Verbesserung der Schaumstabilität eingesetzt. Man erreicht damit, dass der Schaum während der Polymerisation nur geringfügig schrumpft. Als Verdickungsmittel kommen alle hierfür bekannten natürlichen und synthetischen Polymeren in Betracht, die die Viskosität eines wässrigen Systems stark erhöhen und nicht mit den Aminogruppen der basischen Polymeren reagieren. Hierbei kann es sich um wasserquellbare oder wasserlösliche synthetische und natürliche Polymere handeln. Eine ausführliche Übersicht über Verdicker findet man beispielsweise in den Veröffentlichungen von R.Y. Lochhead und W.R. Fron, Cosmetics & Toiletries, 108, 95-135 (Mai 1993) und M.T. Clarke, "Rheological Additives" in D. Laba (ed.) "Rheological Properties of Cosmetics and Toiletries", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polyethylenglykole oder Copolymerisate aus Ethylenglykol und Propylenglykol sowie hochmolekulare Polysaccharide wie Stärke, Guarkernmehl, Johannisbrotkernmehl oder Derivate von Naturstoffen wie Carboxymethylcellulose, Hydroxyethylcellulose, Hydroxymethylcellulose, Hydroxypropylcellulose und Cellulosemischether. Eine weitere Gruppe von Verdickern sind wasserunlösliche Produkte, wie feinteiliges Cellulosepulver oder andere feinteilige Pulver von vernetzten Polymerisaten. Die wässrigen Mischungen können die Verdicker in Mengen bis zu 30 Gew.-% enthalten. Falls solche Verdickungsmittel überhaupt eingesetzt werden, sind sie in Mengen von 0,1, vorzugsweise 0,5 bis 20 Gew.-% in der wässrigen Mischung enthalten.

Um die Schaumstruktur zu optimieren, kann man wahlweise Kohlenwasserstoffe mit mindestens 5 C-Atomen im Molekül zu der wässrigen Reaktionsmischung zusetzen. Geeignete Kohlenwasserstoffe sind beispielsweise Pentan, Cyclopentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wässrigen Mischung während des Schäumens liegt. Die aliphatischen Kohlenwasserstoffe erhöhen die Standzeit der noch nicht polymerisierten geschäumten wässrigen Reaktionsmischung. Dadurch wird das Handling der noch nicht polymerisierten Schäume erleichtert und die Prozesssicherheit erhöht. Die Kohlenwasserstoffe wirken beispielsweise als Zellkeimbildner und stabilisieren gleichzeitig den bereits gebildeten Schaum. Darüber hinaus können sie beim Polymerisieren des Monomerschaums ein weiteres Schäumen der Mischung bewirken. Sie können dann auch die Funktion eines Treibmittels haben. Anstelle von Kohlenwasserstoffen oder in Mischung damit kann man wahlweise auch chlorierte oder fluorierte Kohlenwasserstoffe als Zellkeimbildner und/oder Schaumstabilisator einsetzen, z.B. Dichlormethan, Trichlormethan, 1,2-Dichlorethan, Trichlorfluormethan oder 1,1,2-Trichlortrifluorethan. Falls Kohlenwasserstoffe eingesetzt werden, verwendet man sie beispielsweise in Mengen von 0,1 bis 20 Gew.-%, vorzugsweise 0,1 bis 10 Gew.-%, bezogen auf die polymerisierbare wässrige Mischung.

Die Eigenschaften der Schaumstoffe können wahlweise auch mit Hilfe von weiteren Fasern modifiziert werden. Hierbei kann es sich um natürliche oder synthetische Fasern bzw. um Fasergemische handeln, z.B. Fasern aus Cellulose, Wolle, Polyethylen, Polypropylen, Polyestern oder Polyamiden. Falls Fasern eingesetzt werden, können sie beispielsweise in einer Menge bis zu 200 Gew.-%, vorzugsweise bis zu 25 Gew.-% in der wässrigen Mischung vorhanden sein. Füllstoffe und Fasern können wahlweise auch der bereits geschäumten Mischung zugesetzt werden. Die Mitverwendung von Fasern führt zu einer Erhöhung der Festigkeitseigenschaften wie Nassfestigkeit des fertigen Schaumstoffs. Die Verwendung von Fasern in superabsorbierenden Schäumen ist bekannt.

### Schäume speziell zur Absorption von salzhaltigen wässrigen Lösungen

Um Schäume herzustellen, die auch gegenüber salzhaltigen wässrigen Lösungen ein hohes Absorptionsvermögen aufweisen, setzt man die basischen und die sauren Superabsorber im Gemisch ein, vorzugsweise in nichtneutralisierter Form. Der Neutralisationsgrad der sauren wasserabsorbierenden Polymeren beträgt beispielsweise 0 bis 100, vorzugsweise 0 bis 75 und meistens 0 bis 50 Mol-%. Die wasserabsorbierenden basischen Polymeren haben in Form der freien Basen eine höhere Aufnahmekapazität für salzhaltige wässrige Lösungen und insbesondere saure wässrige Lösungen als in der mit Säuren neutralisierten Form. Wenn basische Polymere als alleinige wasserabsorbierende Polymere eingesetzt werden, so beträgt der Neutralisationsgrad beispielsweise 0 bis 100, vorzugsweise 0 bis 60 Mol-%.

### Herstellung der Schäume

Die oben beschriebenen vernetzbaren wässrigen Mischungen, die die Monomeren oder das basische Polymer, Vernetzer, anorganisches Pulver und Tensid sowie wahlweise weitere Komponenten enthalten, werden zunächst geschäumt. Man kann beispielsweise ein inertes Gas unter einem Druck von z.B. 2 bis 400 bar in der vernetzbaren wässrigen Mischung lösen und sie anschließend auf Atmosphärendruck entspannen. Beim Entspannen aus einer Düse entsteht ein fließfähiger Schaum. Man kann die vernetzbare wässrige Mischung auch nach einer anderen Methode schäumen, indem man darin feine Blasen eines inerten Gases dispergiert. Das Schäumen der vernetzbaren wässrigen Mischung kann im Labor beispielsweise dadurch erfolgen, dass man die wässrige Mischung in einer Küchenmaschine, die mit einem Schneebesen ausgerüstet ist, schäumt. Die Schaumerzeugung wird vorzugsweise in einer Inertgasatmosphäre und mit Inertgasen durchgeführt, z.B. durch Versetzen mit Stickstoff oder Edelgasen unter Normaldruck oder erhöhtem Druck, z.B. bis zu 25 bar und anschließendes Entspannen. Die Konsistenz der Schäume, die Größe der Gasblasen und die Verteilung der Gasblasen im Schaum kann beispielsweise durch die Auswahl der Tenside, Lösevermittler, Schaumstabilisatoren, Zellkeimbildner, Verdickungsmittel und Füllstoffe in einem weiten Bereich variiert werden. Dadurch kann man die Dichte, den Grad der Offenzelligkeit des Schaumstoffs und Wandstärke des Schaumstoffs leicht einstellen. Die wässrige Mischung wird vorzugsweise bei Temperaturen geschäumt, die unterhalb des Siedepunkts der Bestandteile der wässrigen Mischung liegen, z.B. bei Raumtemperatur bis zu 100°C, vorzugsweise bei 20 bis 50°C. Man kann jedoch auch bei Temperaturen oberhalb des Siedepunkts der Komponente mit dem niedrigsten Siedepunkt arbeiten, indem man die Mischung in einem druckdicht verschlossenen Behälter schäumt. Man erhält vernetzbare, schaumförmige Mischungen, die fließfähig und über einen längeren Zeitraum stabil sind. Die Dichte der geschäumten, vernetzbaren Mischung beträgt bei einer Temperatur von 20°C beispielsweise 0,01 bis 0,9 g/cm³.

### Polymerisation und/oder Vernetzen der geschäumten Mischung

In der zweiten Stufe des Verfahrens erfolgt die Polymerisation der Monomeren bzw. die Vernetzung des basischen Polymeren unter Bildung eines wasserabsorbierenden basischen Polymeren. Bei der Polymerisation werden z.B. mindestens zwei ethylenisch ungesättigte Doppelbindungen enthaltende Verbindungen als Vernetzer eingesetzt. Die Polymerisation wird in Gegenwart üblicher Radikale bildender Initiatoren durchgeführt. Man erhält dann vernetzte Polymere, die superabsorbierend sind.

Das ursprünglich wasserlösliche basische Polymer wird durch die Vernetzung wasserunlöslich. Man erhält ein Hydrogel eines basischen Polymers. Die vernetzbaren schaumförmigen Mischungen werden beispielsweise in geeignete Formen transferiert und darin erhitzt, so dass die Monomeren polymerisieren bzw. die Vernetzer mit dem basischen Polymer reagieren. Das geschäumte Material kann beispielsweise in der gewünschten Stärke auf ein temporäres Trägermaterial, das vorteilhafterweise mit einer Antihaftbeschichtung versehen ist, aufgebracht werden. Man kann beispielsweise den Schaum auf eine Unterlage aufrakeln. Eine andere Möglichkeit besteht darin, die schaumförmige wässrige Mischung in Formen einzufüllen, die ebenfalls vorzugsweise antihaftbeschichtet sind. Zur Erzeugung grafischer Zeichen auf der Oberfläche des superabsorbierenden Schaums weist die Form auf mindestens einer Innenfläche grafische Zeichen auf, die sich auf dieser Innenfläche durch eine andere chemische Zusammensetzung oder eine andere physikalische Oberflächenbeschaffenheit als die übrigen Bereiche der Innenfläche von diesen unterschieden.

Da die geschäumte wässrige Mischung eine lange Standzeit aufweist, eignet sich diese Mischung auch für die Herstellung von Verbundmaterialien. Sie kann beispielsweise auf ein permanentes Trägermaterial aufgebracht werden, z.B. Folien aus Polymeren (z.B. Folien aus Polyethylen, Polypropylen oder Polyamid) oder Metallen wie Aluminium. Man kann die geschäumte wässrige Mischung auch auf Vliese, Fluff, Tissues, Gewebe, natürliche oder synthetische Fasern, oder auf andere Schäume auftragen. Bei der Herstellung von Verbundmaterialien kann es unter Umständen vorteilhaft sein, den Schaum in Gestalt von bestimmten Strukturen oder in unterschiedlicher Schichtdicke auf ein Trägermaterial aufzubringen. Es ist jedoch auch möglich, den Schaum auf Fluff-Schichten oder Vliese aufzutragen und diese Materialien so zu imprägnieren, dass der Fluff nach der Vernetzung integraler Bestandteil des Schaums ist. Die in der ersten Verfahrensstufe erhältliche geschäumte wässrige Mischung kann auch zu großen Blöcken geformt und vernetzt werden. Die Blöcke können nach der Vernetzung zu kleineren Formkörpern geschnitten oder gesägt werden. Man kann auch sandwichartige Strukturen herstellen, indem man eine geschäumte wässrige Mischung auf eine Unterlage aufträgt, die schaumförmige Schicht mit einer Folie bzw. Vliesen, Tissues, Geweben, Fasern oder anderen Schäumen abdeckt und die sandwichartige Struktur durch Erhitzen vernetzt. Es ist jedoch auch möglich, vor oder nach dem vernetzen mindestens eine weitere Schicht aus einer geschäumten, vernetzbaren Schicht aufzutragen und wahlweise mit einer weiteren Folie, Vliesen, Tissues, Geweben, Fasern oder anderen Materialien zu bedecken. Der Verbund wird dann in der zweiten Verfahrensstufe der Vernetzung unterworfen. Man kann jedoch auch sandwichartige Strukturen mit weiteren Schaumschichten gleicher oder unterschiedlicher Dichte herstellen.

Erfindungsgemäße Schaumschichten mit einer Schichtdicke von bis zu etwa 1 Millimeter stellt man beispielsweise durch einseitiges Erwärmen oder insbesondere durch einseitiges Bestrahlen der geschäumten polymerisierten bzw. vernetzbaren wässrigen Mischung her. Falls dickere Schichten eines Schaums hergestellt werden sollen, z.B. Schäume mit Dicken von mehreren Zentimetern, ist die Erwärmung des vernetzbaren geschäumten Materials mit Hilfe von Mikrowellen besonders vorteilhaft, weil auf diesem Wege eine relativ gleichmäßige Erwärmung erreicht werden kann. Die Vernetzung erfolgt dabei beispielsweise bei Temperaturen von 20 bis 180°C, vorzugsweise in dem Bereich von 40°C bis 160°C, insbesondere bei Temperaturen von 65 bis 140°C. Bei dickeren Schaumschichten, die vernetzt werden sollen, wird die geschäumte Mischung beidflächig mit Wärme behandelt, z.B. mit Hilfe einer Kontaktheizung oder durch Bestrahlung. Die Dichte des schaumförmigen Hydrogels entspricht im Wesentlichen der Dichte der vernetzbaren wässrigen Mischung. Man erhält somit Schaumstoffe aus wasserabsorbierenden Polymeren mit einer Dichte von beispielsweise 0,01 bis 0,9 g/cm³, vorzugsweise 0,1 bis 0,7 g/cm³. Die schaumförmigen Polymere sind offenzellig. Der Anteil an offenen Zellen beträgt beispielsweise mindestens 80%, vorzugsweise liegt er oberhalb von 90%. Besonders bevorzugt sind Schäume mit einem offenzelligen Anteil von 100%. Der Anteil an offenen Zellen im Schaum wird beispielsweise mit Hilfe der Rasterelektronenmikroskopie (Scanning Electron Microscopy) bestimmt.

Bevorzugt sind Schaumstoffe, die dadurch erhältlich sind, dass man von einer polymerisierbaren wässrigen Mischung ausgeht, die zu mindestens 50% mit Natronlauge oder Kalilauge neutralisierte Acrylsäure, einen mindestens zwei ethylenisch ungesättigte Doppelbindungen enthaltenden Vernetzer, einen Initiator, superabsorbierende Fasern aus einem hydrolysierten und anschließend vernetzten Copolymerisat aus Isobuten und Maleinsäureanhydrid und mindestens ein Tensid enthält. Weitere Beispiele für superabsorbierende Schaumstoffe sind dadurch erhältlich, dass man eine polymerisierbare wässrige Mischung schäumt, die mindestens ein basisches Polymer aus der Gruppe von Vinylamineinheiten enthaltenden Polymeren, Vinylguanidineinheiten enthaltenden Polymeren, Dialkylaminoalkyl(meth)acrylamideinheiten enthaltenden Polymeren, Polyethyleniminen, mit Ethylenimin gepfropften Polyamidoaminen und Polydiallyldimethylammoniumchloriden enthält.

Schaumstoffe mit besonders hoher Wasseraufnahmekapazität und einem verbesserten Aufnahmevermögen für elektrolythaltige wässrige Lösungen sind durch Vernetzen von geschäumten wässrigen Mischungen basischer Polymerisate erhältlich, die, bezogen auf die Polymermischung, 10 bis 90 Gew.-% eines feinteiligen, wasserabsorbierenden, sauren Polymeren enthalten. Das saure Hydrogel kann als festes partikuläres Polymer oder als geschäumtes teilchenförmiges Polymer mit Teilchengrößen von beispielsweise 10 bis 2000 µm in den erfindungsgemäßen Schaumstoffen vorliegen.

Nach dem Vernetzen der geschäumten Mischung oder während des Vernetzens erfolgt die Trocknung des schaumförmigen Hydrogels. Hierbei werden Wasser und andere flüchtige Bestandteile aus dem vernetzten schaumförmigen Hydrogel entfernt. Die Trocknung erfolgt vorzugsweise nach dem Vernetzen des schaumförmigen Hydrogels. Beispiele für geeignete Trocknungsverfahren sind thermische Konvektionstrocknung wie beispielsweise Horden-, Kammer-, Kanal-, Flachbahn-, Teller-, Drehtrommel-, Rieselschacht-, Siebband-, Strom-, Wirbelschicht-, Fließbett-, Schaufel- und Kugelbetttrocknung, thermische Kontakttrocknung wie Heizteller-, Walzen-, Band-, Siebtrommel, Schnecken-, Taumel- und Kontaktscheibentrocknung, Strahlungstrocknung wie beispielsweise Infrarottrocknung, dielektrische Trocknung z.B. Mikrowellentrocknung und Gefriertrocknung. Um unerwünschte Zersetzungs- und Vernetzungsreaktionen zu vermeiden, kann es vorteilhaft sein, die Trocknung bei reduziertem Druck, unter einer Schutzgasatmosphäre und/oder unter schonenden thermischen Bedingungen, bei denen die Produkttemperatur 120°C, bevorzugt 100°C, nicht überschreitet, durchzuführen. Besonders geeignete Trocknungsverfahren stellen die (Vakuum)bandtrocknung und (sofern ein gebrochener Schaum gewünscht oder tolerabel ist) die Schaufeltrocknung dar.

Nach dem Trocknungsschritt enthält das schaumförmige Hydrogel meistens kein Wasser mehr. Der Wassergehalt des geschäumten Materials kann jedoch durch Befeuchten des Schaums mit Wasser oder Wasserdampf beliebig eingestellt werden. Meistens beträgt der Wassergehalt des schaumförmigen Gels 1 bis 60 Gew.-%, vorzugsweise 2 bis 10 Gew.-%. Mit Hilfe des Wassergehalts kann die Flexibilität des schaumförmigen Hydrogels eingestellt werden. Vollständig getrocknete schaumförmige Hydrogele sind hart und spröde, während geschäumte Materialien mit einem Wassergehalt von beispielsweise 5 bis 20 Gew.-% flexibel sind. Die geschäumten Hydrogele können entweder in Form von Folien oder Granulaten direkt verwendet werden oder man schneidet aus dickeren Schaum-Blöcken einzelne Platten oder Folien.

Die oben beschriebenen schaumförmigen Hydrogele können jedoch noch dahingehend modifiziert werden, dass man die Oberfläche der geschäumten Materialien nachvernetzt. Dadurch kann die Gelstabilität der Formkörper aus den geschäumten Hydrogelen verbessert werden. Um eine Oberflächennachvernetzung durchzuführen, behandelt man die Oberfläche der Formkörper aus den geschäumten Hydrogelen mit mindestens einem Vernetzungsmittel, meist in Form einer Lösung, (dem "Oberflächenvernetzer", Nachvernetzer" oder "Oberflächennachvernetzer") und erhitzt die so behandelten Formkörper auf eine Temperatur, bei der diese Vernetzer mit dem noch nicht nachvernetzten superabsorbierenden Schaum reagieren.

Geeignete Nachvernetzungsmittel sind beispielsweise:
- Di- oder Polyepoxide, etwa Di- oder Polyglycidylverbindungen wie Phosphonsäurediglycidylether , Ethylenglykoldiglycidylether oder Bischlorhydrinether von Polyalkylenglykolen,
- Alkoxysilylverbindungen,
- Polyaziridine, Aziridin-Einheiten enthaltende Verbindungen auf Basis von Polyethern oder substituierten Kohlenwasserstoffen, beispielsweise Bis-N-Aziridinomethan,
- Polyamine oder Polyamidoamine sowie deren Umsetzungsprodukte mit Epichlorhydrin,
- Polyole wie Ethylenglykol, 1,2-Propandiol, 1,4-Butandiol, Glycerin, Methyltriglykol, Polyethylenglykole mit einem mittleren Molekulargewicht Mw von 200 - 10000, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis (N-methylol-methacrylamid) oder Melamin-Formaldehyd-Harze,
- Verbindungen mit zwei oder mehr blockierten Isocyanat-Gruppen wie beispielsweise Trimethylhexamethylendüsocyanat blockiert mit 2,2,3,6-Tetramethylpiperidinon-4.

Bei Bedarf können saure Katalysatoren wie beispielsweise p-Toluolsulfonsäure, Phosphorsäure, Borsäure oder Ammoniumdihydrogenphosphat zugesetzt werden.

Besonders geeignete Nachvernetzungsmittel sind Di- oder Polyglycidylverbindungen wie Ethylenglykoldiglycidylether, die Umsetzungsprodukte von Polyamidoaminen mit Epichlorhydrin, 2-Oxazolidinon und N-Hydroxyethyl-2-oxazolidinon.

Die Oberflächennachvernetzung (oft auch nur "Nachvernetzung") wird üblicherweise so durchgeführt, dass eine Lösung des Oberflächennachvernetzers (oft auch nur "Nachvernetzer") auf die Oberfläche des Schaums aufgesprüht wird.

Oberflächennachvernetzer werden vorzugsweise in Form einer wasserhaltigen Lösung auf die Schaumstoffoberfläche aufgebracht. Die wasserhaltige Lösung kann wassermischbare organische Lösemittel enthalten, z.B. Alkohole wie Methanol, Ethanol und/oder i-Propanol oder Ketone wie Aceton. Die Menge an Vernetzer, die auf die Oberfläche der schaumförmigen Hydrogele aufgetragen wird, beträgt beispielsweise 0,1 bis 5 Gew.-%, vorzugsweise 1 bis 2 Gew.-%. Die Oberflächennachvernetzung der schaumförmigen Hydrogele erfolgt durch Erhitzen der mit mindestens einem Vernetzer behandelten schaumförmigen Hydrogele bei einer Temperatur von beispielsweise 60 bis 120°C, vorzugsweise bei 70 bis 100°C. Nach der Oberflächenvernetzung kann der Wassergehalt-der geschäumten, an der Oberfläche nachvernetzten Hydrogele ebenfalls auf Werte von 1 bis 60 Gew.-% eingestellt werden.

Nachbehandlung der Schäume mit Komplexierungsmittel und/oder Quellverzögerungsmittel

### Komplexierungsmittel

In einer Ausführung der Erfindung wird die Stabilität des Schaums, vorzugsweise eines Schaums, der aus einer säuregruppentragende Monomeren enthaltenden polymerisierbaren Mischung erzeugt wurde, durch Ausbildung von Komplexen auf der Schaumoberfläche erhöht. Die Bildung der Komplexe auf dem Schaum erfolgt durch Behandeln mit mindestens einem Komplexierungsmittel. Ein Komplexierungsmittel ist ein Mittel, das komplexierende Kationen enthält. Vorzugsweise wird dies durch Aufsprühen von Lösungen zwei- oder mehrwertiger Kationen bewirkt, wobei die Kationen mit funktionellen Gruppen, etwa den Säuregruppen des polymeren Schaums unter Ausbildung von Komplexen reagieren können. Beispiele für zwei- oder mehrwertige Kationen sind formal ganz oder teilweise aus Vinylaminmonomeren aufgebaute Polymere wie teilweise oder vollständig hydrolysiertes Polyvinylamid (sogenanntes "Polyvinylamin"), dessen Amingruppen stets - auch bei sehr hohen pH-Werten - teilweise zu Ammoniumgruppen protoniert vorliegen oder Metallkationen wie Mg²⁺, Ca^{2*}, Al³⁺, Sc³⁺, Ti⁴⁺, Mn²⁺, Fe^{2+/3+}, Co²⁺, Ni²⁺, Cu²⁺, Zn²⁺, Y³⁺, Zr⁴⁺, La³⁺, Ce⁴⁺, Hf⁴⁺, und Au^{3*}. Bevorzugte Metall-Kationen sind Mg²⁺, Ca²⁺, A1³⁺, Ti⁴⁺, Zr⁴⁺ und La³⁺, und besonders bevorzugte Metall-Kationen sind Al³⁺, Ti⁴⁺ und Zr⁴⁺. Die Metall-Kationen können sowohl allein als auch im Gemisch untereinander eingesetzt werden. Die Anionen unterliegen keiner wesentlichen Einschränkung, es sind von den genannten Metall-Kationen alle solchen Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat, Hydrogensulfat, Carbonat, Hydrogencarbonat, Nitrat, Phosphat, Hydrogenphosphat, Dihydrogenphosphat und Carboxylat, wie Acetat und Lactat. In besonders bevorzugter Form wird Aluminiumsulfat Al₂(SO₄)₃ verwendet. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Bespiel Wasser/Methanol, Wasser/1,2-Propandiol und Wasser/1,3-Propandiol. Ganz besonders bevorzugt ist Wasser.

Die Konzentration des mehrwertigen Metallions in der wässrigen Lösung beträgt im Allgemeinen mindestens 1 Gew.-%, vorzugsweise mindestens 2 Gew.-% sowie im Allgemeinen höchstens 20 Gew.-%, vorzugsweise höchstens 10 Gew.-%. Eingesetzt werden im Allgemeinen mindestens 0,05 Gew.-%, vorzugsweise mindestens 0,1 Gew.-%, in besonders bevorzugter Weise mindestens 0,2 Gew.-%, beispielsweise mindestens 0,8 Gew.-% sowie im Allgemeinen höchstens 10 Gew.-%, vorzugsweise höchstens 8 Gew.-% und in besonders bevorzugter Weise höchstens 5 Gew.-%, beispielsweise höchstens 3,2 Gew.-%, des mehrwertigen Metallions bezogen auf den trocknen Schaum vor Aufbringung von Komplexierungs- oder Quellverzögerungsmittel. "Trocken" im Rahmen dieser Erfindung ist ein Schaum, der 5 Gew.-% Wasser enthält. Bei Verwendung von Aluminiumsulfat entspricht ein Gehalt von 0,8 Gew.-% des Kations einem Gehalt von Al₂(SO₄)₃ von 5 Gew.-% und ein Gehalt von 3,2 Gew.-% des Kations einem Gehalt von Al₂(SO₄)₃ von 20 Gew.-%.

### Quellverzögerungsmittel

Wahlweise wird der Schaum mit mindestens einem Quellverzögerungsmittel behandelt. Unter Quellverzögerungsmittel werden Stoffe verstanden, die auf die Oberfläche des superabsorbierenden Schaums im feuchten oder trockenen Zustand (<10 Gew.-% Wasser im Schaum) aufgebracht werden und zu einer Verzögerung der Flüssigkeitsaufnahme führen, was in manchen Anwendungen vorteilhaft sein kann. Die Behandlung von Superabsorbern mit Quellverzögerungsmitteln ist bekannt.

### Verwendung der Schäume

Die erfindungsgemäßen schaumförmigen Hydrogele, die wahlweise an der Oberfläche nachvernetzt sind, können für alle Zwecke verwendet werden, für die beispielsweise die aus der EP 858 478 B1 bekannten wasserabsorbierenden, schaumförmigen Hydrogele auf Basis von Säuregruppen enthaltenden Polymeren wie vernetzten Polyacrylaten, eingesetzt werden. Die erfindungsgemäßen schaumförmigen Hydrogele eignen sich beispielsweise für den Einsatz in Hygieneartikeln zur Absorption von Körperflüssigkeiten, in Verbandmaterial zur Abdeckung von Wunden, als Dichtungsmaterial, als Verpackungsmaterial, als Bodenverbesserungsmittel, als Bodenersatzstoff, zur Entwässerung von Schlämmen, zur Absorption saurer wässriger Abfälle, zum Eindicken wässriger Lacke bei der Entsorgung von Restmengen an Lacken, zur Entwässerung von wasserhaltigen Ölen oder Kohlenwasserstoffen oder als Material für Filter in Lüftungssystemen.

Ein weiterer Gegenstand der Erfindung sind Hygieneartikel, welche die erfindungsgemäßen Schäume enthalten, wie Babywindeln, Damenbinden, Inkontinenzartikel und Verbandmaterial. Die erfindungsgemäßen Schäume erfüllen beispielsweise in Hygieneartikeln mehrere Funktionen, nämlich Akquisition, Distribution und/oder Speicherung von Körperflüssigkeiten. Insbesondere eignen sie sich zur Speicherung von Körperflüssigkeiten als Storage Layer in einer Windel oder einem Damenhygieneartikel. Durch die Verzögerung der Flüssigkeitsaufnahme wird die zu absorbierende Flüssigkeit erst gleichmäßig in dem Hygieneartikel verteilt. So kann bei einer zeitlich versetzten zweiten oder mehrfachen Beaufschlagung mit Flüssigkeit die gesamte Fläche des Schaums weiterhin in besseren Maße zur Verfügung stehen.

Konstruktionen von Hygieneartikeln, die absorbierende Schaumschichten enthalten, sind bekannt, ebenso ihre Herstellung. Der erfindungsgemäßen absorbierenden Schaumstoffe werden in diesen Hygieneartikeln ebenso wie bekannte absorbierende Schaumstoffe eingesetzt. Schaumstoffschichten aus den erfindungsgemäß schaumförmigen Hydrogelen können beispielsweise in einer Stärke von 1 bis 5 mm in einem der obengenannten Hygieneartikel als absorbierender Kern zwischen einer oberen flüssigkeitsdurchlässigen Abdeckung und einer unteren flüssigkeitsundurchlässigen Schicht aus einer Folie aus z.B. Polyethylen oder Polypropylen angeordnet sein. Die flüssigkeitsdurchlässige Schicht des Hygieneartikels steht bei der Anwendung in direktem Kontakt mit der Haut des Anwenders. Dieses Material besteht üblicherweise aus einem Vlies aus natürlichen Fasern wie Cellulosefasern oder Fluff. Wahlweise ist oberhalb und/oder unterhalb des absorbierenden Kerns noch eine Tissueschicht angeordnet. Zwischen der untern Schicht des Hygieneartikels und dem absorbierenden Kern kann wahlweise noch eine Speicherschicht aus einem herkömmlichen partikulären anionischen Superabsorber vorhanden sein. Wenn man die geschäumten basischen Hydrogele als absorbierenden Kern in Windeln einsetzt, so kann aufgrund der offenzelligen Struktur der geschäumten basischen Hydrogele die normalerweise in einzelnen Mengen auf einmal beaufschlagte Körperflüssigkeit zügig abgeführt werden. Dadurch wird dem Anwender ein angenehmes Gefühl der Oberflächentrockenheit der Windel vermittelt.

### Bestimmungsmethoden

### Zentrifugenretentionskapazität ("Centrifuge Retention Capacity", "CRC")

Bei dieser Methode wird das Retentionsvermögen des Superabsorberschaums im Teebeutel gegen Schwerkraft in g Flüssigkeit pro g Superabsorberschaum bestimmt. Zur Bestimmung der CRC werden 0,2000 +/- 0,0050 g des zu prüfenden Superabsorberschaums in einen 60 x 85 mm großen Teebeutel eingeführt, der anschließend verschweißt wird. Idealerweise ist der Schaum trocken, d.h. wasserfrei, da Restfeuchte die Wasseraufnahme des Schaums und damit die CRC entsprechend absenkt. Nachdem die CRC jedoch typischerweise ein Wert von mindestens 3 g/g ist und oft auch deutlich höher liegt, führt ein geringer Wassergehalt des Schaums nicht zu merklichen Messfehlern. Wassergehalte bis 5 Gew.-%, bezogen auf das Gewicht des feuchten Schaums, sind ohne Weiteres tolerabel, meist auch höhere Wassergehalte. Die gemessenen CRC-Werte können bei Bedarf auch um den Wassergehalt des Schaums rechnerisch korrigiert werden.

Der Teebeutel wird für 30 Minuten in einen Überschuss von 0,9 Gew.-%iger Kochsalz-lösung gegeben (mindestens 0,83 I Kochsalz-Lösung / 1 g Polymer). Anschließend wird der Teebeutel 3 Minuten lang bei 250 g zentrifugiert. Die Bestimmung der absorbierten und auch gegen Schwerkraft zurückgehaltenen Flüssigkeitsmenge geschieht durch Auswägen des zentrifugierten Teebeutels. In gleicher Weise wird mit einem nicht mit Superabsorberschaum gefüllten Teebeutel ein Blindwert bestimmt, der von der mit Superabsorberschaum bestimmten absorbierten und zurückgehaltenen Flüssigkeitsmenge abgezogen wird. Bei der hier verwendeten Einwaage ergibt sich die CRC einfach durch Multiplikation des so um den Blindwert korrigierten Werts mit 5.

### Wassergehalt

Der Wassergehalt wird nach der von der EDANA (European Disposables and Nonwovens Association, Avenue Eugène Plasky 157, 1030 Brüssel, Belgien; www.edana.org) empfohlenen und von dort erhältlichen Methode 430.2-02 bestimmt.

### Beispiele

### Herstellung der in den Beispielen verwendeten Schäume

In einem Becherglas wurden mit Hilfe eines Magnetrührers die folgenden Komponenten vermischt:

| | |
|---|---|
| 209,13 g | Acrylsäure |
| 81,31 g | einer 37,3 %igen Natriumacrylatlösung in Wasser |
| 16,8 g | Polyethylenglycoldiacrylat-400 |
| 25,60 g | einer 15 %igen, wässrigen Lösung eines Additionsprodukts von 80 Mol Ethylenoxid an 1 mol eines linearen, gesättigten C₁₆-C₁₈ Fettalkohols |
| 26,62 g | Wasser sowie |

die unten in der Tabelle angegebenen Mengen der unten angegebenen anorganischen Feststoffpulver.

Zu dieser Suspension wurden unter Eiskühlung 240,54g Triethanolamin langsam zugegeben und danach auf 15°C abkühlen gelassen. Die erhaltene Lösung wurde in einen Druckbehälter überführt und dort für 25 min bei einem Druck von 12 bar durch Durchleiten eines Kohlendioxidstroms von 300 l/h mit Kohlendioxid gesättigt. Unter Druck wurden 16 g einer 3 Gew.-%igen, wässrigen Lösung von 2,2'-Azobis(2-amidinopropan)dihydrochlorid zugegeben und anschließend wurde für weitere 5 min Kohlendioxid durch die Reaktionsmischung geleitet. Die Reaktionsmischung wurde danach bei einem Druck von 12 bar durch eine Düse mit einem Durchmesser von 1,0 mm ausgepresst, wobei sich ein feinzelliger, gut fließfähiger Schaum bildete.

Der erhaltene Monomerschaum wurde auf eine DIN A3 große Glasplatte mit 3 mm hohen Rändern aufgebracht, die zuerst mit einer silikonisierten Polyesterfolie (silikonisierte Polyethylenterephthalatfolie Hostaphan® RN, 36 µm Dicke, erhältlich von Mitsubishi Polyester Film GmbH, Wiesbaden, Deutschland) bedeckt worden war. Der Schaum wird oben mit einem Stück Folie des gleichen Typs bedeckt, auf das mit einem handelsüblichen Stift zur Beschriftung von Kunststoffen ("Folienstift") grafische Zeichen (ein Muster) aufgebracht worden war. Die obere Folie wird mit einer zweiten Glassplatte bedeckt. Die Schaumprobe wurde synchron von beiden Seiten über 4 Minuten mit UV-Licht bestrahlt, von oben mit einem UV/VIS - Strahler UVASPOT 1000/T der Firma Dr. Hönle AG, Gräfelfing, Deutschland, von unten mit 2 UV/VIS-Strahlern UVASPOT 400/T desselben Herstellers.

Die erhaltene Schaumschicht wurde in einem Vakuumtrockenschrank bei 80°C vollständig unter einem Stickstoffstrom getrocknet und anschließend durch Besprühen mit Wasser auf eine Feuchte von 5 Gew.-% eingestellt.

Die Ergebnisse mit unterschiedlichen Füllstoffen und Füllstoffmengen sind in der folgenden Tabelle zusammengefasst. Die Menge des Füllstoffs im Schaum wurde auf trockenen Schaum umgerechnet. Die Ausbildung grafischer Zeichen auf der Schaumoberfläche wurde optisch bewertet. In der Spalte "Grafik" ist vermerkt, wie gut grafische Zeichen wiedergegeben wurden:
nb nicht bestimmt
0 keine Ausbildung grafischer Zeichen
0+ schwache Ausbildung grafischer Zeichen
+ gute Ausbildung grafischer Zeichen
++ sehr gute Ausbildung grafischer Zeichen

| Beispiel # | Füllstoff | | | Grafik |
|---|---|---|---|---|
| | Art | Menge in Monomerlösung [g] | Menge im Schaum [Gew.-%] | |
| 1 | Kaolin | 4,8 | 1 | 0+ |
| 2 | " | 24 | 4,6 | 0+ |
| 3 | " | 48 | 8,7 | + |
| 4 | " | 96 | 16,1 | + |
| 5 | " | 96 | 16,1 | ++ |
| 6 | Talkum | 4,8 | 1 | nb |
| 7 | " | 24 | 4,6 | nb |
| 8 | " | 48 | 8,7 | 0 |
| 9 | " | 96 | 16,1 | 0 |
| 10 | " | 192 | 27,7 | 0+ |
| 11 | TiO₂ | 4,8 | 1 | 0+ |
| 12 | " | 24 | 4,6 | ++ |
| 13 | " | 48 | 8,7 | ++ |

Die Beispiele zeigen, dass abhängig vom Feststoffgehalt und der Feststoffart Schäume erhalten werden, die an ihrer Oberfläche grafische Zeichen tragen.

## Patentansprüche

1. Superabsorbierender Schaum, der mindestens 1 Gew.-%, bezogen auf das Gesamtgewicht des trockenen Schaums, anorganischen pulverförmigen Feststoffs enthält und an mindestens einer seiner Oberflächen grafische Zeichen trägt, erhältlich durch ein Verfahren, bei dem eine geschäumte wässrige Mischung, die
mindestens 1 Gew.-%, bezogen auf das Gesamtgewicht des trockenen Schaums, anorganischen pulverförmigen Feststoffs
sowie polymerisier- und vernetzbare, wahlweise (teil-)neutralisierte, Säuregruppen enthaltende monoethylenisch ungesättigte Monomere oder mindestens ein vernetzbares basisches Polymer,
sowie Vernetzer, anorganischen pulverförmigen Füllstoff und mindestens ein Tensid
enthält, in einer Form polymerisiert wird, die auf mindestens einer Innenfläche grafische Zeichen trägt.

2. Schaum nach Anspruch 1, der mindestens 5 Gew.-%, bezogen auf das Gesamtgewicht des trockenen Schaums, anorganischen Feststoffs enthält.

3. Schaum nach Anspruch 1 oder 2, der ein vernetztes Polymer auf Basis Säuregruppen tragender Monomere ist.

4. Schaum nach Anspruch 3, der ein vernetztes Polymer auf Basis eines Gemisches aus Acrylsäure und Natriumacrylat ist.

5. Schaum nach einem der Ansprüche 1 bis 4, **dadurch gekennzeichnet, dass** seine Oberfläche nachvernetzt und/oder mit einem Komplexierungsmittel behandelt ist.

6. Schaum nach einem der Ansprüche 1 bis 5, **dadurch gekennzeichnet, dass** der anorganische pulverförmige Feststoff Titandioxid ist.

7. Verfahren zur Herstellung eines superabsorbierenden Schaums, der mindestens 1 Gew.-%, bezogen auf das Gesamtgewicht des trockenen Schaums, anorganischen pulverförmigen Feststoffs enthält und an mindestens einer seiner Oberflächen grafische Zeichen trägt., bei dem eine geschäumte wässrige Mischung, die
mindestens 1 Gew.-%, bezogen auf das Gesamtgewicht des trockenen Schaums, anorganischen pulverförmigen Feststoffs
sowie polymerisier- und vernetzbare, wahlweise (teil-)neutralisierte, Säuregruppen enthaltende monoethylenisch ungesättigte Monomere oder mindestens ein vernetzbares basisches Polymer,
sowie Vernetzer, anorganischen pulverförmigen Füllstoff und mindestens ein Tensid
enthält, in einer Form polymerisiert wird, die auf mindestens einer Innenfläche grafische Zeichen trägt

8. Verfahren nach Anspruch 7, **dadurch gekennzeichnet, dass** man nach der Polymerisation die Oberfläche des Schaums mit einem Oberflächennachvernetzungsmittel und/oder einem Komplexierungsmittel behandelt.

9. Verwendung des in einem der Ansprüche 1 bis 6 definierten superabsorbierenden Schaums in Hygieneartikeln zur Absorption von Körperflüssigkeiten

10. Hygieneartikel zur Absorption von Körperflüssigkeiten, **dadurch gekennzeichnet, dass** er den in den Ansprüchen 1 bis 6 definierten Schaum enthält.

## Claims

1. A superabsorbent foam which comprises at least 1 % by weight, based on the total weight of the dry foam, of inorganic pulverulent solid and bears graphics on at least one of its surfaces, obtainable by a process in which a foamed aqueous mixture which comprises
at least 1% by weight, based on the total weight of the dry foam, of inorganic pulverulent solid,
and polymerizable and crosslinkable, optionally (partly) neutralized, monoethylenically unsaturated monomers comprising acid groups or at least one crosslinkable basic polymer,
and also crosslinkers, inorganic pulverulent filler and at least one surfactant,
is polymerized in a mold which bears graphics on at least one internal surface.

2. The foam according to claim 1, which comprises at least 5% by weight, based on the total weight of the dry foam, of inorganic solid.

3. The foam according to claim 1 or 2, which is a crosslinked polymer based on monomers bearing acid groups.

4. The foam according to claim 3, which is a crosslinked polymer based on a mixture of acrylic acid and sodium acrylate.

5. The foam according to any of claims 1 to 4, whose surface has been postcrosslinked and/or treated with a complexing agent.

6. The foam according to any of claims 1 to 5, wherein the inorganic pulverulent solid is titanium dioxide.

7. A process for producing a superabsorbent foam which comprises at least 1 % by weight, based on the total weight of the dry foam, of inorganic pulverulent solid and bears graphics on at least one of its surfaces, in which a foamed aqueous mixture which comprises
at least 1 % by weight, based on the total weight of the dry foam, of inorganic pulverulent solid,
and polymerizable and crosslinkable, optionally (partly) neutralized, monoethylenically unsaturated monomers comprising acid groups or at least one crosslinkable basic polymer,
and also crosslinkers, inorganic pulverulent filler and at least one surfactant,
is polymerized in a mold which bears graphics on at least one internal surface.

8. The process according to claim 7, wherein the polymerization is followed by treatment of the surface of the foam with a surface postcrosslinking agent and/or a complexing agent.

9. The use of the superabsorbent foam defined in any of claims 1 to 6 in hygiene articles for absorbing body fluids.

10. A hygiene article for absorbing body fluids, which comprises the foam defined in claims 1 to 6.

## Revendications

1. Mousse superabsorbante, qui contient au moins 1 % en poids, par rapport au poids total de la mousse sèche, de matière solide inorganique pulvérulente et porte des signes graphiques sur au moins une de ses surfaces, pouvant être obtenue par un procédé dans lequel on polymérise un mélange aqueux expansé qui contient
au moins 1 % en poids, par rapport au poids total de la mousse sèche, de matière solide inorganique pulvérulente
ainsi que des monomères à insaturation monoéthylénique, contenant des groupes acides, polymérisables et réticulables, au choix (partiellement) neutralisés, ou au moins un polymère basique réticulable,
ainsi que des agents de réticulation, une charge inorganique pulvérulente et au moins un tensioactif,
dans un moule qui porte des signes graphiques sur au moins une surface interne.

2. Mousse selon la revendication 1, qui contient au moins 5 % en poids, par rapport au poids total de la mousse sèche, de matière solide inorganique.

3. Mousse selon la revendication 1 ou 2, qui est un polymère réticulé à base de monomères portant des groupes acides.

4. Mousse selon la revendication 3, qui est un polymère réticulé à base d'un mélange d'acide acrylique et d'acrylate de sodium.

5. Mousse selon l'une quelconque des revendications 1 à 4, **caractérisée en ce que** sa surface est post-réticulée et/ou traitée par un complexant.

6. Mousse selon l'une quelconque des revendications 1 à 5, **caractérisée en ce que** la matière solide inorganique pulvérulente est du dioxyde de titane.

7. Procédé pour la production d'une mousse superabsorbante, qui contient au moins 1 % en poids, par rapport au poids total de la mousse sèche, de matière solide inorganique pulvérulente et porte des signes graphiques sur au moins une de ses surfaces, dans lequel on polymérise un mélange aqueux expansé qui contient
au moins 1 % en poids, par rapport au poids total de la mousse sèche, de matière solide inorganique pulvérulente
ainsi que des monomères à insaturation monoéthylénique, contenant des groupes acides, polymérisables et réticulables, au choix (partiellement) neutralisés, ou au moins un polymère basique réticulable,
ainsi que des agents de réticulation, une charge inorganique pulvérulente et au moins un tensioactif,
dans un moule qui porte des signes graphiques sur au moins une surface interne.

8. Procédé selon la revendication 7, **caractérisé en ce qu'**après la polymérisation on traite la surface de la mousse par un agent de post-réticulation superficielle et/ou un complexant.

9. Utilisation de la mousse superabsorbante définie dans l'une quelconque des revendications 1 à 6, dans des articles d'hygiène destinés à l'absorption de liquides corporels.

10. Article d'hygiène destiné à l'absorption de liquides corporels, **caractérisé en ce qu'**il contient la mousse définie dans les revendications 1 à 6.
